# EUROPÄISCHE PATENTSCHRIFT

(11) **EP 0 923 917 B1**
(45) Veröffentlichungstag und Bekanntmachung des Hinweises auf die Patenterteilung: **26.11.2003**
(21) Anmeldenummer: 98122874.5
(22) Anmeldetag: 02.12.1998
(51) Int. Cl.: A61F 5/055

(54) **Cervicalstütze**
Cervical collar
Collier cervical

(30) Priorität: 17.12.1997 DE 19755923
(43) Veröffentlichungstag der Anmeldung: 23.06.1999
(73) Patentinhaber: Beiersdorf Aktiengesellschaft, 20245 Hamburg (DE)
(72) Erfinder: Herzberg, Thorsten, 21149 Hamburg (DE); Möller, Thomas, 20457 Hamburg (DE); Kartheus, Holger, 20255 Hamburg (DE); Andrews, Arthur-Hugh, 25337 Kölln-Reisiek (DE)

(56) Entgegenhaltungen:
- WO-A-87/01028
- DE-A- 3 906 232
- DE-U- 8 119 906
- US-A- 3 850 164
- US-A- 5 275 581

## Beschreibung

Die Erfindung betrifft eine medizinische Halskrawatte mit dreidimensionaler Struktur zur Führung, Stützung, Immobilisierung und Sicherung der Halswirbelsäule, bestehend im wesentlichen aus einem formstabilen Stützkörper aus elastischem Schaumstoff, der um den Hals des Patienten gelegt wird und dessen freie Enden sich im Bereich des Nacken überlappen und lösbar miteinander verbunden sind, dessen obere und untere Konturlinie der anatomischen Form des Kinnbereiches beziehungsweise des Schulterbereiches des Patienten angepaßt sind, wobei gegebenenfalls der Stützkörper mit einem Überzug aus einem anschmiegsamen Textilmaterial versehen ist. Des weiteren wird ein Verfahren zur Herstellung der Halskrawatte offenbart.

Medizinische Halskrawatten, auch Cervicalstützen genannt, weisen das folgende Indikationsfeld auf.

Eine Verwendung dieser ist beim Cervikalsyndrom und beim Schleudertrauma angeraten. Des weiteren können Cervicalstützen in der postoperativen Rehabilitation nach Operationen im Hals-Wirbelsäulenbereich oder an der Bandscheibe vorteilhaft eingesetzt werden. Dann kann eine Fraktur der Halswirbelsäule stabilisiert werden.
Neben den aufgeführten existieren aber eine weitere Vielzahl von Einsatzgebieten für bekannte Cervicalstützen.

Die im allgemein bekannten Bandagen für diesen Indikationsbereich besitzen meist eine Konstruktion aus einem Schaumstoff. Es sind auch Halskrawatten in einer Kombinationskonstruktion aus Schaumstoff mit Kunststoffversteifungen bekannt. Diese Art der Kombinationskonstruktion sind von einer festen Struktur und finden im üblichen für ein anderes Indikationsfeld Verwendung, zum Beispiel frische Frakturen im Hals-Wirbelsäulenbereich.

So beschreibt die DE-PS 24 04 683 eine Cervicalstütze, die einen formstabilen Stützkörper aus elastischem Schaumstoff aufweist, der um den Hals des Patienten gelegt wird und dessen freie Enden sich im Bereich des Nacken überlappen und lösbar miteinander verbunden sind, dessen obere und untere Konturlinie der anatomischen Form des Kinnbereiches beziehungsweise des Schulterbereiches des Patienten angepaßt sind. Die hier offenbarte Cervicalstütze hat sich zwar in der Praxis gut bewährt, ist aber an individuelle Anforderungen nicht anpaßbar.

In der DE 39 06 232 A1 wird eine Cervicalstütze offenbart, die ebenfalls einen um den Hals des Patienten legbaren Schaumstoffstützkörper aufweist. Um die Cervicalstütze an die jeweiligen Erfordernisse der vorliegenden Indikation anzupassen, wird erfindungsgemäß vorgeschlagen, in dem Stützkörper zumindest einen Durchbruch vorzusehen, durch den ein Stützelement geführt wird, das einen Schaft sowie einen großflächigen, am Innenumfang des Stützkörpers zur Anlage kommenden und diesen dort versteifenden Kopf aufweist. Gegebenenfalls können mehrere Stützelemente durch ein flexibles Band miteinander verankert werden.
Ein derartig geformtes Stützelement muß aber, um die gestellte Aufgabe zu erfüllen, aus einem flexiblen Kunststoffmaterial halbsteifen Charakters sein, insbesondere wenn die Stützelemente mit einem Band verbunden werden sollen.

Aufgabe der Erfindung war es daher, eine Halskrawatte zur Verfügung zu stellen, die eine offene, leichte Konstruktion aufweist, ein Schwitzen oder Wärmestau auf ein Minimum reduziert und eine hohe Therapiesicherheit bei einer funktionellen Konstruktion zeigt.

Gelöst wird diese Aufgabe durch eine Halskrawatte, wie sie in Anspruch 1 dargelegt ist. Gegenstand der Unteransprüche sind dabei vorteilhafte Weiterbildungen der Halskrawatte.

Demgemäß betrifft die Erfindung eine medizinische Halskrawatte, bestehend im wesentlichen aus einem formstabilen Stützkörper aus elastischem Schaumstoff, der um den Hals des Patienten gelegt wird und dessen freie Enden sich im Bereich des Nackens überlappen und lösbar miteinander verbunden sind, dessen obere und untere Konturlinie der anatomischen Form des Kinnbereiches beziehungsweise des Schulterbereiches des Patienten angepaßt sind, wobei gegebenenfalls der Stützkörper mit einem Überzug aus einem anschmiegsamen Textilmaterial versehen ist. Erfindungsgemäß ist in dem Stützkörper zumindest ein Stabilisierungselement aus offenzelligem Schaumstoff, aus geschlossenzelligem Schaumstoff oder aus Vliesstoff derartig angeordnet, daß es den Stützkörper radial durchdringt und von diesem vollständig umgeben ist.

Die Stabilisierungselemente weisen einen Körper aus Schaumstoff auf, auf den eine Deckschicht und/oder eine Innenlage insbesondere aus einem weichen Schaumstoff oder Vlies aufgebracht sind, die den Körper im Kantenbereich überragen, vorzugsweise um 2 mm bis 6 mm.

In einer vorteilhaften Ausführungsform weisen die Stabilisierungselemente einen Körper auf, der eine kreisförmige, längsovale, ellipsenförmige oder länglich abgerundete Form hat oder eine rechteckige Form, dessen Kanten abgerundet sind.

Die Innenlage bietet den besonderen Vorteil, daß je nach Ausführungsform eine partielle Friktion ausgeübt werden kann. Die Innenlage kann aber auch derartig gestaltet sein, daß ein besonders weicher Übergang von der Basis des jeweilgen Stabilisierungselementes zum umgebenden Stützkörper gewährleistet wird.

Durch diese Konstruktion ist es möglich, die Stabilisierungselemente am äußeren Bereich mit dem Stützkörper der Halskrawatte zu verkleben, um keine störenden Verbindungsstellen zum inneren Bereich zu haben. Außerdem deckt der Überstand der Abdeckung die Verbindungsstelle nach außen ab.
Bevorzugt werden dabei Hot-Melt-Klebeverfahren angewendet, wobei handelsübliche Kleber wie zum Beispiel Kontaktkleber oder Silikonkleber Verwendung finden können, aber auch der Einsatz von doppelseitigen Klebebändern hat sich als geeignet erwiesen.

In einer weiteren bevorzugte Ausführungsform sind die Stabilisierungselemente in Sandwichbauweise aufgebaut, bestehend aus mehreren Lagen unterschiedlichen Materials, insbesondere Schaumstoff.

Dann hat es sich als vorteilhaft gezeigt, wenn in jedem Seitenbereich des Stützkörpers jeweils ein Stabilisierungselement angeordnet ist, dessen Körper einen im wesentlichen ellipsoiden Querschnitt aufweist.

Die Stabilisierungselemente sollten jeweils im posterioren Bereich gegenüber dem anterioren Bereich verdickt ausgeführt sein.

Schließlich kann die oben dargelegte medizinische Halskrawatte besonders vorteilhaft nach folgendem Verfahren hergestellt werden, und zwar wird
a) der Stützkörper aus einem Schaumstoffblock ausgeschnitten oder ausgestanzt und im Nackenbereich mit einem Verschlußsystem versehen,
b) in den Stützkörper werden die Ausnehmungen für die Stabilisierungselemente eingefügt,
c) die Körper der Stabilisierungselemente werden aus einem Schaumstoffblock ausgeschnitten oder ausgestanzt, wobei das Schneiden auch thermisch mit bekannter Technologie erfolgen kann,
d) auf die Körper wird jeweils die Deckschicht aufgeklebt,
e) die Stabilisierungselemente werden in die Ausnehmungen eingefügt,
f) die Halskrawatte wird gegebenenfalls mit einem Überzug aus dem anschmiegsamen Textilmaterial überzogen.

Der vorteilhafte offenzellige Schaumstoff, aus dem der Stützkörper der Halskrawatte geformt ist, ermöglicht die Aufnahme von Schweiß und weist einen hervorragenden Polstereffekt auf, was zu einer Reduzierung von Druckstellen im Halsbereich des Patienten führt. Dies ist insofern wichtig, da gerade im anterioren Bereich Druck auf den Kehlkopf, auf das Sternum, auf die Clavicula und die Unterkieferpartie zu vermeiden ist.

Durch die erfindungsgemäße Integration von Stabilisierungselementen kann eine definierte erhöhte Funktionssicherung im Sinne von Stabilität, Führung und Fixierung der Halswirbelsäule erreicht werden.

Da die Stabilisierungselemente austauschbar sind, können je nach Anwendungsfall Stabilisierungselemente mit einer angepaßten höheren oder niedrigeren Festigkeit eingesetzt werden.

In Sandwichbauweise hergestellte Stabilisierungselemente haben den großen Vorteil, daß durch unterschiedliche Festigkeiten der verwendeten Materialien, Stabilisierungselemente mit unterschiedlichen Charakter auf der Innen- und/der Außenseite beziehungsweise im Kernbereich der Halskrawatte individuell eingesetzt werden können. hergestellt.

Im folgenden soll eine besonders vorteilhafte Ausführung der erfindungsgemäßen Halskrawatte mittels mehrerer Figuren beschrieben werden, ohne damit die Erfindung unnötig einschränken zu wollen.

Im einzelnen zeigen
- die Figur 1: eine Halskrawatte, die mit mehreren Stabilisierungselementen versehen ist,
- die Figur 2: ein besonders vorteilhaft geformtes Stabilisierungselement in der Draufsicht,
- die Figur 3: das besonders vorteilhaft geformte Stabilisierungselement in der seitlichen Ansicht, wobei zwei Ausführungsformen gezeigt sind,
- die Figur 4: eine zweite Ausführungsform der Halskrawatte, in der zwei der vorteilhaften Stabilisierungselemente integriert sind.

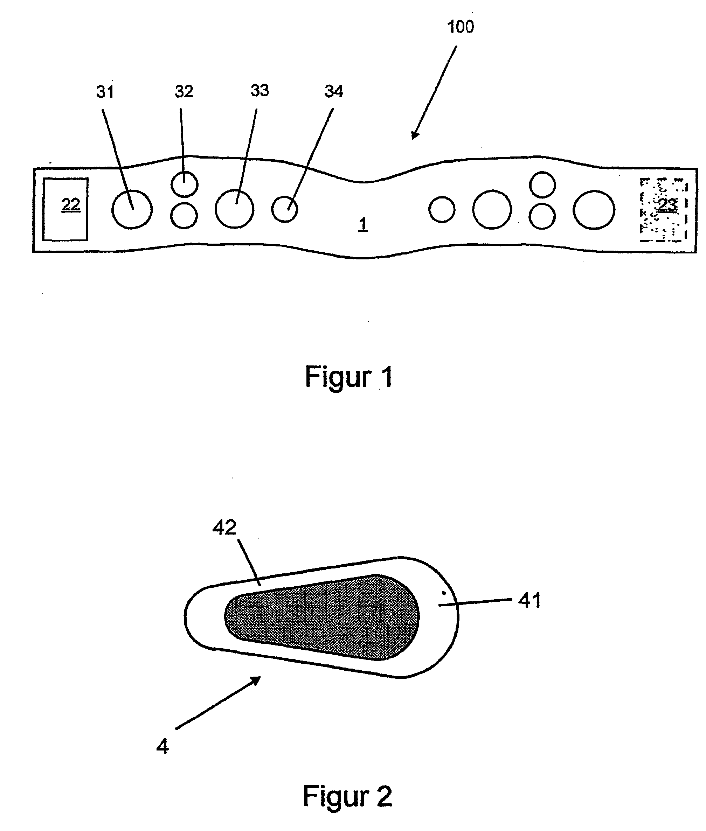

In der Figur 1 ist die Halskrawatte 100 dargestellt. Die Halskrawatte 100 besteht im wesentlichen aus einem formstabilen Stützkörper 1. Dieser Stützkörper 1 wird um den Hals des Patienten gelegt. Die freien Enden des Stützkörpers 1 überlappen sich im Bereich des Nackens und werden dort mittels eines Klettverschlusses, dessen Unterteil 21 auf der oberen Seite des Stützkörpers 1 angebracht, insbesondere aufgenäht ist, und dessen Oberteil 22, das auf der Unterseite bevorzugt vernäht ist, miteinander lösbar verbunden.
In dem Stützkörper 1 sind eine Vielzahl von Ausschnitten 31, 32, 33, 34 vorhanden. In die Ausschnitte 31, 32, 33, 34 werden entsprechend geformte Stabilisierungselemente 4 eingefügt, die bevorzugt eine größere Härte aufweisen als der sie umgebende Stützkörper 1. Es hat sich als vorteilhaft erwiesen, wenn die Ausschnitte 31, 32, 33, 34 symmetrisch zu beiden Seiten des Halses vorgesehen sind. Im Bedarfsfalle ist aber durchaus möglich, unterschiedliche Anordnungen oder Formen der Ausschnitte 31, 32, 33, 34 auf den Seiten vorzusehen oder auch Stabilisierungselemente 4 mit differierenden Härtegraden einzusetzen.

Die Figur 2 zeigt ein besonders vorteilhaft geformtes Stabilisierungselement 4. Das Stabilisierungselement 4 setzt sich aus zwei Lagen zusammen. Die obere Lage, die Deckschicht 41, besteht aus einem weichen Schaumstoff, der die zweite Lage des Stabilisierungselements 4, den Körper 42, im Kantenbereich um hier 4 mm überragt. Der Körper 42 ist aus einem relativ harten Schaumstoff geformt, um in der Halskrawatte 100 eine stützende Funktion übernehmen zu können. Die nockenförmig geformte Deckschicht 31 ist auf den ebenfalls nockenförmig hergestellten Körper 42 aufgeklebt.

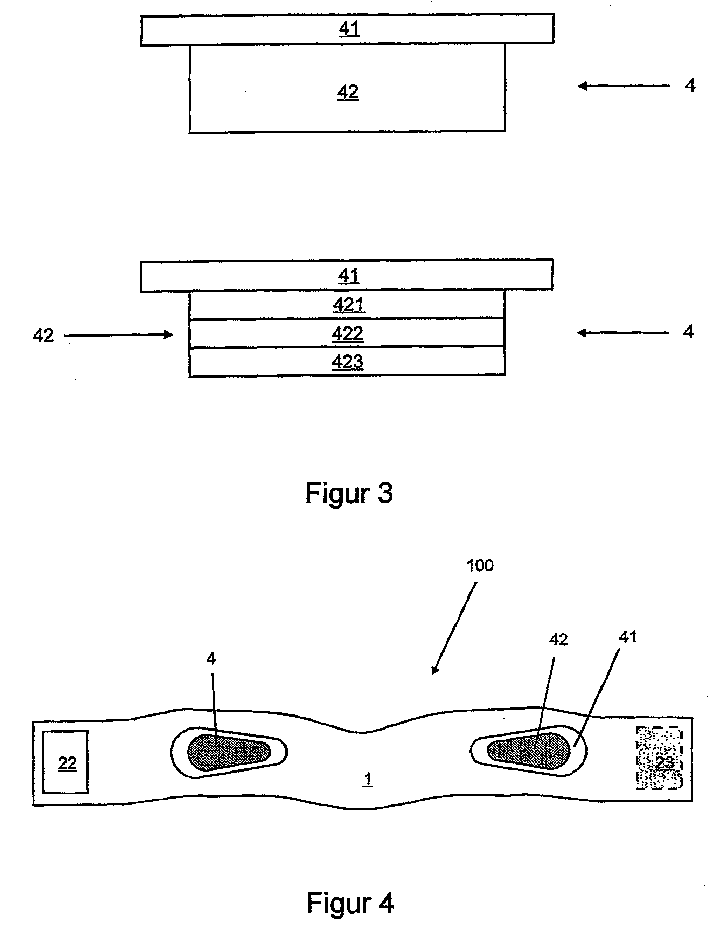

In der Figur 3 ist in der seitlichen Ansicht nochmals das Stabilisierungselement aus der Figur 2 gezeigt. Die auf den Körper 42 geklebte Deckschicht 41 überragt diesen. Der Körper 42 kann aus einer einzigen Schicht von Schaumstoff bestehen, es ist aber auch möglich, je nach Anwendungsfall den Körper 42 in Sandwichbauweise auszuführen, wobei die unterschiedlichen Lagen 421, 422, 423 des Sandwichs durchaus verschiedene Eigenschaften aufweisen können. Die Herstellung des Sandwichs erfolgt beispielsweise dadurch, daß die einzelnen Lagen 421, 422, 423 miteinander verklebt werden.

In der Figur 4 schließlich ist eine zweite, besonders geeignete Ausführungsform der Halskrawatte 100 dargestellt. Im Stützkörper 1 der Halskrawatte 100 sind zwei nockenförmige Stabilisierungselemente 4 vorhanden, wie sie in den Figuren 2 und 3 offenbart werden. Die Stabilisierungselemente 4 werden dabei mit dem Körper 42 in die vorhandenen in dem Stützkörper 1 vorhandenen Ausschnitte 31, 32 eingeführt, wobei die Ausschnitte 31, 32 annähernd die gleichen Abmessungen aufweisen wie die Körper 42. Die überragende Deckschicht 41 verbleibt auf der Außenseite des Stützkörpers 1. Zur sicheren Fixierung können die Deckschichten 41 auf dem Stützkörper verklebt werden, ein nachträgliches Verrutschen ist damit ausgeschlossen. Die Stabilisierungselemente 4 sind dabei derartig in das Stützelement 1 eingefügt, daß die Stabilisierungselemente 4 jeweils im posterioren Bereich gegenüber dem anterioren Bereich verdickt ausgeführt sind.

Wie auch schon in Figur 1 ist der vorzugsweise vorhandene Überzug aus Textil für den Stützkörper 1 nicht dargestellt.

## Patentansprüche

1. Medizinische Halskrawatte, bestehend aus einem formstabilen Stützkörper (1) aus elastischem Schaumstoff, der um den Hals des Patienten gelegt wird und dessen freie Enden sich im Bereich des Nackens überlappen und lösbar miteinander verbunden sind, dessen obere und untere Konturlinie der anatomischen Form des Kinnbereiches beziehungsweise des Schulterbereiches des Patienten angepasst sind, wobei in dem Stützkörper (1) zumindest ein Stabilisierungselement (4) derartig angeordnet ist, dass es den Stützkörper (1) radial durchdringt und von diesem vollständig umgeben ist, **dadurch gekennzeichnet, dass**
die Stabilisierungselemente (4) aus offenzelligem Schaumstoff oder aus geschlossenzelligem Schaumstoff bestehen und die Stabilisierungselemente einen Körper (42) aus Schaumstoff aufweisen, auf den eine Deckschicht (41) und/oder eine Innenlage aufgebracht sind, die den Körper im Kantenbereich überragen.

2. Medizinische Halskrawatte nach Anspruch 1, **dadurch gekennzeichnet, daß**
die Stabilisierungselemente (4) einen Körper (42) aufweisen, der eine kreisförmige, längsovale, ellipsenförmige oder länglich abgerundete Form aufweist oder eine rechteckige Form, dessen Kanten abgerundet sind.

3. Medizinische Halskrawatte nach Anspruch 1, **dadurch gekennzeichnet, daß**
die Deckschicht (41) den Körper (42) im Kantenbereich um 2 mm bis 6 mm überragt.

4. Medizinische Halskrawatte nach Anspruch 1, **dadurch gekennzeichnet, daß**
die Stabilisierungselemente (4) in Sandwichbauweise aufgebaut sind, bestehend aus mehreren Lagen unterschiedlichen Materials, insbesondere Schaumstoff.

5. Medizinische Halskrawatte nach Anspruch 1, **dadurch gekennzeichnet, daß**
in jedem Seitenbereich des Stützkörpers (1) jeweils ein Stabilisierungselement (4) angeordnet, dessen Körper (42) ein im wesentlichen ellipsoiden Querschnitt aufweist.

6. Medizinische Halskrawatte nach Anspruch 1, **dadurch gekennzeichnet, daß**
die Stabilisierungselemente (4) jeweils im posterioren Bereich gegenüber dem anterioren Bereich verdickt ausgeführt sind.

7. Medizinische Halskrawatte nach Anspruch 1, **dadurch gekennzeichnet, daß**
der Stützkörper (1) mit einem Überzug aus einem anschmiegsamen Textilmaterial versehen ist.

8. Medizinische Halskrawatte nach Anspruch 1, **dadurch gekennzeichnet, daß** die eine Deckschicht (41) und/oder Innenlage aus einem weichen Schaumstoff oder Vlies besteht.

9. Medizinische Halskrawatte nach Anspruch 1, **dadurch gekennzeichnet, daß** die Stabilisierungselemente (4) anstelle aus Schaumstoff aus Vliesstoff bestehen.

10. Verfahren zur Herstellung einer medizinischen Halskrawatte nach mindestens einem der vorhergehenden Ansprüche 1 bis 8, wobei
a) der Stützkörper (1) aus einem Schaumstoffblock ausgeschnitten oder ausgestanzt wird und im Nackenbereich mit einem Verschlusssystem (22, 23) versehen wird,
b) in den Stützkörper (1) die Ausnehmungen (31, 32, 33, 34) für die Stabilisierungselemente (4) eingefügt werden,
c) die Körper (42) der Stabilisierungselemente (4) aus einem Schaumstoffblock ausgeschnitten oder ausgestanzt werden,
d) auf die Körper (42) jeweils die Deckschicht (41) beziehungsweise die Innenlagen aufgeklebt werden,
e) die Stabilisierungselemente (4) in die Ausnehmungen (31, 32, 33, 34) eingefügt werden,
f) gegebenenfalls die Halskrawatte mit einem Überzug aus dem anschmiegsamen Textilmaterial überzogen wird.

## Claims

1. Medical neck collar, consisting of a shape-stable support body (1) made of elastic foam which is placed around the patient's neck and whose free ends overlap and are releasably connected to one another in the area of the back of the neck, and whose upper and lower contour lines are adapted to the anatomical form of the patient's chin area and shoulder area, respectively, at least one stabilizing element (4) being arranged in the support body (1) in such a way that it passes radially through the support body (1) and is surrounded completely by the latter, **characterized in that** the stabilizing elements (4) are made of open-cell foam or of closed-cell foam and the stabilizing elements have a body (42) of foam onto which a top layer (41) and/or an inside layer are/is applied, extending beyond the body in the edge area.

2. Medical neck collar according to Claim 1, **characterized in that** the stabilizing elements (4) have a body (42) which has a circular, oval, ellipsoid or long and rounded shape or has a rectangular shape whose edges are rounded.

3. Medical neck collar according to Claim 1, **characterized in that** the top layer (41) extends beyond the body (42) in the edge area by 2 mm to 6 mm.

4. Medical neck collar according to Claim 1, **characterized in that** the stabilizing elements (4) are built up in a sandwich-style structure consisting of several layers of different material, in particular foam.

5. Medical neck collar according to Claim 1, **characterized in that** in each side area of the support body (1) there is a stabilizing element (4) whose body (42) has a substantially ellipsoid cross section.

6. Medical neck collar according to Claim 1, **characterized in that** the stabilizing elements (4) are each made thicker in the posterior region compared to the anterior region.

7. Medical neck collar according to Claim 1, **characterized in that** the support body (1) is provided with a covering made of a conformable textile material.

8. Medical neck collar according to Claim 1, **characterized in that** the top layer (41) and/or inside layer are/is made of a soft foam or nonwoven.

9. Medical neck collar according to Claim 1, **characterized in that** the stabilizing elements (4) are made of a nonwoven material instead of foam.

10. Method for producing a medical neck collar according to at least one of the preceding Claims 1 to 8, in which method:
a) the support body (1) is cut or punched out from a block of foam and is provided with a closure system (22, 23) in the area for the back of the neck,
b) the recesses (31, 32, 33, 34) for the stabilizing elements (4) are formed in the support body (1),
c) the bodies (42) of the stabilizing elements (4) are cut or punched out from a block of foam,
d) the top layer (41) and the inside layers are respectively adhesively bonded onto the bodies (42) ,
e) the stabilizing elements (4) are inserted into the recesses (31, 32, 33, 34),
f) if appropriate, the neck collar is covered with a covering of the conformable textile material.

## Revendications

1. Collier cervical médical, se composant d'un corps de support de forme stable (1) en mousse élastique, qui est placé autour du cou du patient et dont les extrémités libres se recouvrent dans la région de la nuque et sont raccordées de manière détachable l'une à l'autre, dont les lignes de contour supérieure et inférieure sont adaptées à la forme anatomique de la région du menton ou respectivement de la région des épaules du patient, au moins un élément de stabilisation (4) étant disposé dans le corps de support (1) de telle sorte qu'il traverse radialement le corps de support (1) et qu'il soit complètement entouré par celui-ci, **caractérisé en ce que**
les éléments de stabilisation (4) se composent d'une mousse à cellules ouvertes ou de mousse à cellules fermées et les éléments de stabilisation présentent un corps (42) en mousse sur lequel sont placées une couche de recouvrement (41) et/ou une couche interne, qui dépassent du corps dans la région des bords.

2. Collier cervical médical selon la revendication 1, **caractérisé en ce que** les éléments de stabilisation (4) présentent un corps (42) qui présente une forme circulaire, ovale oblongue, ellispoïdale ou longitudinale arrondie, ou une forme rectangulaire, dont les bords sont arrondis.

3. Collier cervical médical selon la revendication 1, **caractérisé en ce que** la couche de recouvrement (41) dépasse du corps (42) de 2 mm à 6 mm dans la région des bords.

4. Collier cervical médical selon la revendication 1, **caractérisé en ce que** les éléments de stabilisation (4) sont construits en sandwich, se composant de plusieurs couches de matériau différent, en particulier en mousse.

5. Collier cervical médical selon la revendication 1, **caractérisé en ce que** dans chaque région latérale du corps de support (1) est disposé à chaque fois un élément de stabilisation (4) dont le corps (42) présente une forme en section transversale essentiellement ellipsoïde.

6. Collier cervical médical selon la revendication 1, **caractérisé en ce que** les éléments de stabilisation (4) sont réalisés à chaque fois dans la région postérieure de manière épaissie par rapport à la région antérieure.

7. Collier cervical médical selon la revendication 1, **caractérisé en ce que** le corps de support (1) est pourvu d'un revêtement en un matériau textile épousant les formes.

8. Collier cervical médical selon la revendication 1, **caractérisé en ce que** la couche de recouvrement (41) et/ou la couche interne se compose d'une mousse souple ou d'un voile.

9. Collier cervical médical selon la revendication 1, **caractérisé en ce que** les éléments de stabilisation (4) se composent d'un matériau en voile au lieu de mousse.

10. Procédé de fabrication d'un collier cervical médical selon au moins l'une des revendications 1 à 8 précédentes, dans lequel
a) le corps de support (1) est découpé ou estampé dans un bloc de mousse et est pourvu dans la région de la nuque d'un système de fermeture (22, 23),
b) dans le corps de support (1) sont pratiqués les évidements (31, 32, 33, 34) pour les éléments de stabilisation (4),
c) les corps (42) des éléments de stabilisation (4) sont découpées ou estampé dans un bloc de mousse,
d) la couche de recouvrement (41), respectivement les couches internes sont collées à chaque fois sur le corps (42),
e) les éléments de stabilisation (4) sont introduits dans les évidements (31, 32, 33, 34),
f) éventuellement le collier cervical médical est recouvert d'un revêtement en le matériau textile épousant les formes.
